# FASCICULE DE BREVET EUROPEEN

(11) **EP 4 031 078 B1**
(45) Date de publication et mention de la délivrance du brevet: **05.04.2023**
(21) Numéro de dépôt: 20820257.2
(22) Date de dépôt: 17.09.2020
(51) Int. Cl.: A61F 5/01, A61F 5/058

(54) **ATTELLE ACTIVE POUR TRAITEMENT D'UNE SUBLUXATION DU PÉRONÉ ET KIT LA COMPRENANT**
AKTIVE SCHIENE ZUR BEHANDLUNG EINER PERONEUS-SUBLUXATION UND KIT MIT DIESER SCHIENE
ACTIVE SPLINT FOR TREATMENT OF PERONEAL SUBLUXATION AND KIT INCLUDING IT

(30) Priorité: 20.09.2019 FR 1910378
(43) Date de publication de la demande: 27.07.2022
(73) Titulaire: Lève-toi et Marche, 34280 La Grande Motte (FR)
(72) Inventeur: ASTIER, Thierry, 34280 La Grande Motte (FR)
(74) Mandataire: Ipside
(86) Numéro de dépôt international: PCT/FR2020/051608
(87) Numéro de publication internationale: WO 2021/053299

(56) Documents cités:
- US-A- 2 172 484
- US-A1- 2012 271 215
- US-A1- 2016 151 192

## Description

### Domaine technique de l'invention

L'invention s'inscrit dans le domaine des appareils médicaux. Elle relève des appareils de traitement et/ou rééducation notamment d'un membre inférieur d'un utilisateur, et concerne plus particulièrement une attelle active permettant en particulier de corriger la position osseuse du péroné qui a subi une subluxation et un kit comportant ladite attelle active.

L'attelle est dite « active » dans le sens où elle est destinée à agir activement sur l'utilisateur et en particulier sur son membre inférieur. US 2016/151192 A1 est considéré comme l'état de la technique le plus proche.

### Technique antérieure

Le péroné, aussi appelé « fibula » dans la nouvelle nomenclature, constitue la partie latérale du squelette de la jambe. Il est articulé à ses extrémités distale et proximale avec le tibia et avec son extrémité distale avec le talus.

Le péroné peut, dans certains cas de dysfonctionnement, par son extrémité proximale, subir un mouvement ascensionnel à l'origine d'une décoaptation articulaire entraînant une subluxation du péroné.

Par ailleurs, l'ascension de l'articulation péronéo-tibiale supérieure entraîne l'ascension de l'articulation péronéo-tibiale inférieure et provoque la réduction de la surface de coaptation entre le talus et le péroné. Cette situation est généralement à l'origine d'un diastasis entre le péroné et le tibia et/ou entre le péroné et le talus.

En outre, une subluxation du péroné peut entraîner la distension du rétinaculum des extenseurs du pied. Dans ce cas, les muscles et leurs tendons releveurs du pied et notamment le tibial antérieur sont inefficaces dans leur rôle du maintien du système tenon-mortaise formé par le talus, le tibia et le péroné.

Notamment, une subluxation du péroné risque de générer d'autres pathologies chez la personne atteinte, par exemple, suite à un phénomène de compensation des vertèbres, il peut apparaître des lombalgie, sciatique, crurale, lumbago, hernie discale...

Le traitement d'une subluxation est généralement réalisé par l'intervention d'un professionnel médical et consiste en une manipulation de la jambe de la personne atteinte afin de normaliser les articulations péronéo-tibiales supérieure et inférieure.

Ce traitement est réalisé tout au long d'une série de séances de manipulations. Le dysfonctionnement sur la jambe concernée est progressivement corrigé à chaque séance de manipulation mais réapparaît partiellement entre deux séances successives, jusqu'à sa disparition totale.

Il apparaît par conséquent un besoin de traiter la subluxation de manière continue, de sorte à éviter au patient la douleur accompagnant cette pathologie entre deux séances consécutives de manipulation.

Une solution pour assurer le maintenir des surfaces articulaires péronéo-tibiale supérieure et inférieure de façon continue et sans risque de récidive, est de normaliser manuellement le péroné, puis de faire une contention par plâtre ou résine.

Toutefois, cette solution n'est pas satisfaisante dans la mesure où elle nécessite l'immobilisation d'une grande partie de la jambe, ce qui peut compliquer la marche et générer des difficultés dans les déplacements de la personne à traiter.

En outre, le traitement de la subluxation est incomplet car cela ne compense pas la composante ascensionnelle et décoaptatrice du biceps fémoral qui est hypo-extensible.

Compte-tenu de ce qui précède, un autre besoin pour la correction d'une subluxation du péroné réside dans le fait de pouvoir se passer de praticien et de rendre cette correction à la portée de la personne atteinte.

### Présentation de l'invention

La présente invention a pour objectif de palier les inconvénients précités.

À cet effet, l'invention concerne une attelle active notamment pour le traitement d'une subluxation du péroné, comportant un fourreau dans lequel un coulisseau est agencé mobile en coulissement entre une position rétractée et une position déployée, le coulisseau comprenant une tête destinée à reposer en appui contre la tête d'un péroné d'un utilisateur, ladite attelle active comprenant :
- des moyens de rappel élastiques configurés pour solliciter le coulisseau vers sa position rétractée,
- un organe de blocage du coulisseau adapté à maintenir ledit coulisseau dans sa position déployée,
- des moyens de détente adaptés à agir sur l'organe de blocage de sorte à libérer le coulisseau de sa position déployée, cette libération provoquant l'entraînement du coulisseau en position rétracté sous l'effet des moyens de rappel élastique.

Lors de sa course, la tête du coulisseau, qui maintien une pression constante sur la tête du péroné, provoque la normalisation de la position de cette dernière.

La présente invention permet donc, grâce à son action correctrice, de supprimer les douleurs articulaires, musculaires, osseuses, nerveuses dorsales et circulatoires de l'utilisateur, et permet de corriger d'éventuels troubles connexes résultants de la subluxation du péroné, tout en pouvant s'affranchir de toute manipulation d'un praticien.

La présente invention permet également de compenser la composante ascensionnelle et décoaptatrice du biceps fémoral dans la mesure où lorsque l'utilisateur est en position assise, l'attelle compense l'action décoaptatrice du biceps fémoral par application d'une force radiale contre la tête du péroné de l'utilisateur, et lors du passage de l'utilisateur en position debout, l'attelle s'oppose à la composante ascensionnelle grâce aux sollicitations des moyens de rappel élastiques.

Dans des modes particuliers de réalisation, l'invention répond en outre aux caractéristiques suivantes, mises en oeuvre séparément ou en chacune de leurs combinaisons techniquement opérantes.

Dans des modes particuliers de réalisation de l'invention, les moyens de détente agissent sur l'organe de blocage par la déformation élastique du fourreau et du coulisseau, ou uniquement du coulisseau.

Dans des modes particuliers de réalisation de l'invention, l'organe de blocage comprennent une languette élastique formée dans le coulisseau, comportant à une extrémité libre une excroissance adaptée à se loger dans une lumière réalisée dans le fourreau.

Cet organe de blocage a l'avantage d'être simple de conception et de fabrication, ce qui joue favorablement sur la fiabilité du fonctionnement de l'attelle active. Par ailleurs, ces caractéristiques permettent de participer à la réduction des dimensions de l'attelle active.

Dans des modes particuliers de réalisation de l'invention, le fourreau comporte un renfoncement s'étendant en vis-à-vis du coulisseau, ledit renfoncement étant configuré pour recevoir l'excroissance lorsque le coulisseau est en position rétractée et sur une partie de sa course vers sa position déployée, le renfoncement et la lumière étant séparés par un pont de matière.

Cette déformation élastique participe à donner un transmettre un retour tactile à un utilisateur avant le blocage du coulisseau dans sa position déployée.

Dans des modes particuliers de réalisation de l'invention, les moyens de détente sont formés par une partie en porte-à-faux du fourreau, s'étendant au-delà de la lumière, configurée pour être en regard de la languette élastique lorsque le coulisseau est en position déployée et présentant des propriétés élastiques.

Ces moyens de détente ont l'avantage d'être simple de conception et de fabrication, ce qui joue favorablement sur la fiabilité du fonctionnement de l'attelle active. Par ailleurs, ces caractéristiques permettent de participer à la réduction des dimensions de l'attelle active.

Dans des modes particuliers de réalisation de l'invention, le fourreau comporte un flasque intrados destiné à être en appui contre un membre inférieur d'un utilisateur et un flasque extrados opposé au flasque intrados, lesdits flasques étant assemblés l'un avec l'autre et étant configurés pour définir un volume interne dans lequel est engagé le coulisseau.

Dans des modes particuliers de réalisation de l'invention, l'attelle active comporte des organes de guidage permettant le guidage du coulisseau en translation au sein du fourreau, lesdits organes de guidage étant formés par des nervures s'étendant sur le coulisseau et sur le fourreau et coopérant les unes avec les autres.

Dans des modes particuliers de réalisation de l'invention, les moyens de rappel élastique sont formés par au moins un ressort hélicoïdal enfermé dans une cage formée par les organes de guidage, et donc par des nervures.

Selon un autre aspect, la présente invention concerne également un kit de traitement d'une subluxation du péroné comportant une attelle active telle que décrite précédemment et une attelle passive destinée à maintenir la tête du péroné dans une position normalisée, ladite attelle passive comportant :
- deux zones d'appui reliées entre elles par un pont de matière, une des zones d'appui étant destinée à être appliquée contre ladite tête du péroné et l'autre zone d'appui étant destinée à être appliquée contre le fut osseux du péroné,
- un lien élastique destinée à maintenir un effort appliqué par lesdites zones d'appui contre le membre inférieur d'un l'utilisateur.

Selon un autre aspect, la présente invention concerne une attelle active ou un kit tels que décrits précédemment dans son application pour le traitement d'une subluxation du péroné.

### Brève description des figures

L'invention sera mieux comprise à la lecture de la description suivante, donnée à titre d'exemple nullement limitatif, et faite en se référant aux figures suivantes :
[Fig. 1] la figure 1 représente une vue en perspective de l'attelle active selon l'invention comprenant un coulisseau en position rétractée ;
[Fig. 2] la figure 2 représente une vue en perspective d'un coulisseau de l'attelle active de la figure 1 ;
[Fig. 3] la figure 3 représente une autre vue en perspective du coulisseau de la figure 2 ;
[Fig. 4] la figure 4 représente une vue en perspective d'un flasque extrados d'un fourreau de l'attelle active de la figure 1 ;
[Fig. 5] la figure 5 représente une vue en perspective d'un flasque intrados d'un fourreau de l'attelle active de la figure 1 ;
[Fig. 6] la figure 6 représente une vue en perspective de l'attelle active de la figure 1 dans laquelle le coulisseau est en position déployée ;
[Fig. 7] la figure 7 représente une vue en perspective de l'attelle active de la figure 6, dans laquelle le flasque intrados est représenté en transparence.
[Fig. 8] la figure 8 représente une vue en perspective de l'attelle active de la figure 1, dans laquelle le flasque intrados est représenté en transparence.
[Fig. 9] la figure 9 représente une vue en perspective d'une attelle passive d'un kit de traitement d'une subluxation du péroné.

Dans ces figures, des références numériques identiques d'une figure à l'autre désignent des éléments identiques ou analogues. Par ailleurs, pour des raisons de clarté, les dessins ne sont pas à l'échelle, sauf mention contraire.

### Description des modes de réalisation

La présente invention vise une attelle active 10 destinée à déplacer la tête du péroné d'un utilisateur pour la ramener dans sa position normalisée notamment suite à une subluxation.

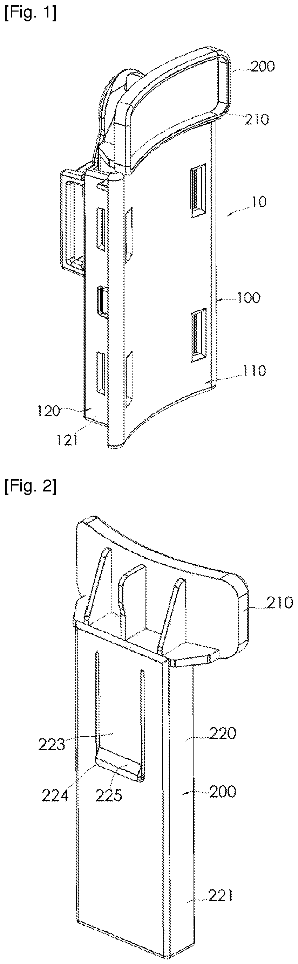

Comme le montre la figure 1, l'attelle active 10 comporte un fourreau 100 avec lequel coopère en coulissement un coulisseau 200 selon un axe de translation, entre une position déployée et une position rétractée.

Le fourreau 100 et le coulisseau 200 s'étendent suivant un même axe longitudinal parallèle ou confondu avec l'axe de translation du coulisseau 200.

L'attelle 10 est destinée à être plaquée latéralement, directement ou indirectement, contre les téguments de la jambe de l'utilisateur en maintenant de façon continue une pression contre celle-ci et plus particulièrement contre le péroné de l'utilisateur. Le péroné étant lié au tibia, cet effort est également transmis au tibia par l'intermédiaire du péroné et permet de corriger le dysfonctionnement lié à la subluxation comme décrit plus en détail ci-après.

À cet effet, l'attelle active 10 peut comporter un lien (non représenté sur les figures), préférentiellement élastique, destiné à entourer la jambe de l'utilisateur afin de maintenir l'attelle contre celle-ci à une pression constante d'intensité prédéfinie. Le lien comporte par exemple deux extrémités libres dont chacune comporte des organes de fixation destinés à coopérer l'un avec l'autre. Ces organes de fixation peuvent être formés par des crochets et boucles textiles.

Dans l'exemple préféré de réalisation de l'invention représenté sur les figures 1 et 4 à 8, le fourreau 100 est formé par deux flasques ou demi-coques, respectivement appelées « flasque intrados » 110 et « flasque extrados » 120, le flasque intrados 110 étant destiné à être fixé contre le membre inférieur d'un utilisateur et le flasque extrados 120 étant destiné à être agencé en vis-à-vis de l'environnement extérieur.

Les flasques intrados 110 et extrados 120 sont configurés pour former un volume interne recevant le coulisseau 200 et comprennent respectivement une face interne et une face externe, lesdites faces externes étant orientées vers l'environnement extérieur et les faces internes étant orientées vers le volume interne.

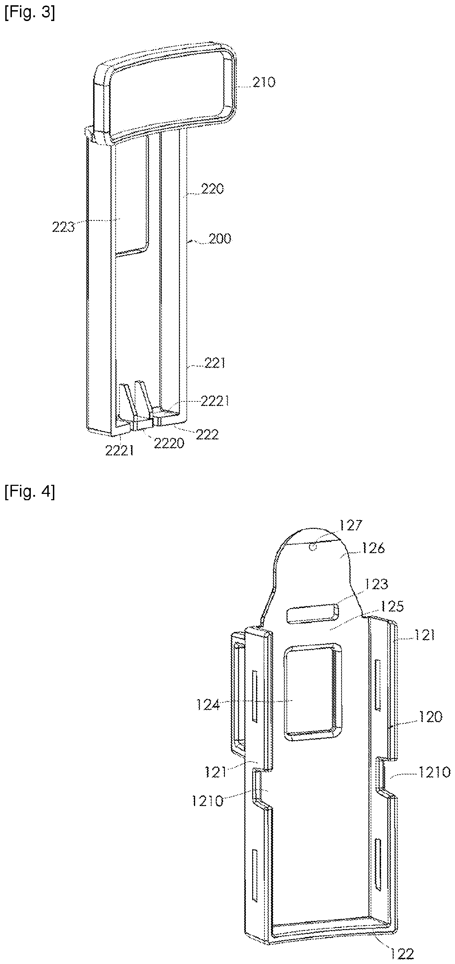

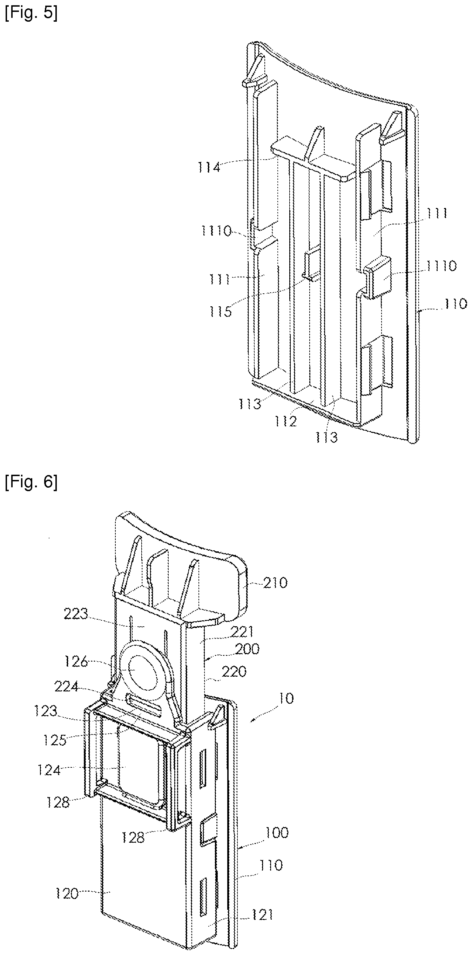

Plus précisément, comme visible notamment sur les figures 4 et 5, afin de définir le volume interne, les flasques intrados 110 et extrados 120 comportent respectivement deux parois latérales parallèles constituant des flancs 111 et 121, s'étendant depuis leur face interne. Les flancs 111 du flasque intrados 110 sont configurés pour coopérer avec les flancs 121 du flasque extrados 120 par emboitage élastique.

Dans l'exemple de réalisation représenté sur les figures 1 et 4 à 8, les flancs 121 du flasque extrados 120 peuvent comprendre des encoches 1210 dans lesquelles sont destinées à venir se loger des protubérances 1110 s'étendant depuis la face interne du flasque intrados 110. Avantageusement, l'imbrication des protubérances 1110 avec les encoches 1120 constitue un détrompeur facilitant l'assemblage des flasques intrados 110 et extrados 120 l'un avec l'autre.

Comme le montrent les figures 4 et 5, les flancs 111 du flasque intrados 110 sont reliés l'un à l'autre par une paroi de fond 112, transversale. De manière analogue, les flancs 121 du flasque extrados 120 sont reliés l'une à l'autre par une paroi de fond 122.

Dans l'exemple de réalisation de l'invention représenté sur les figures 1 et 6 à 8, Les flancs 111 du flasque intrados 110 sont agencés en vis-à-vis du volume interne et les flancs 121 du flasque extrados 120 sont agencés en vis-à-vis du volume externe lorsque le fourreau est assemblé.

La face externe du flasque intrados 110 est destinée à être appliqué contre l'utilisateur et présente avantageusement à cet effet une section droite de forme concave.

La face externe du flasque extrados 120 comprend avantageusement deux boucles 128 à travers le lien destiné à entourer la jambe de l'utilisateur est engagé.

Dans l'exemple de réalisation représenté sur les figures 1 et 4 à 8, le coulisseau 200 est logé entre les flancs 111 et 121 des flasques intrados 110 et extrados 120.

Il présente la forme d'une pièce mince et comporte une tête 210 reliée à un corps 220.

Comme le montre les figures 1 et 6 à 8, le corps 220 du coulisseau 200 est engagé dans le volume interne du fourreau 100 et sa tête 210 s'étend à l'extérieur dudit fourreau 100.

Dans le présent texte, on désigne par les termes « extrémité interne » du coulisseau 200, l'extrémité libre du corps 220 du coulisseau 200.

La tête 210 du coulisseau 200 est destinée à être appliquée directement ou indirectement contre les téguments d'un patient, et plus particulièrement contre la tête de péroné de l'utilisateur.

La tête 210 du coulisseau 200 peut avantageusement présenter une section droite de forme concave, par exemple selon un rayon sensiblement identique à la section droite de la face externe du flasque intrados 110, de sorte à épouser la forme de la jambe du patient. La tête 210 du coulisseau 200 et plus particulièrement sa surface destinée à être en vis-à-vis de la jambe de l'utilisateur peut être recouverte d'une couche de mousse dense, par exemple fixée par collage.

Le coulisseau 200 comporte une face dite « face intrados » destinée à être orientée vers la jambe de l'utilisateur, opposée à une face dite « face extrados » destinée à être orientée vers l'environnement extérieur.

L'attelle active 10 comporte avantageusement des organes de guidage permettant de guider le coulisseau 200 en translation au sein du fourreau 100.

Avantageusement, ces organes de guidage peuvent être formés par des nervures s'étendant sur le coulisseau 200 et sur le fourreau 100, lesdites nervures du coulisseau 200 étant destinées à coopérer avec celles du fourreau 100.

Les figures 2, 3, 6 à 8 présentent un exemple de réalisation dans lequel ces organes de guidage sont notamment formés par deux parois latérales 221 parallèles se faisant face et s'étendant le long du corps 220 du coulisseau 200, de part et d'autre de celui-ci.

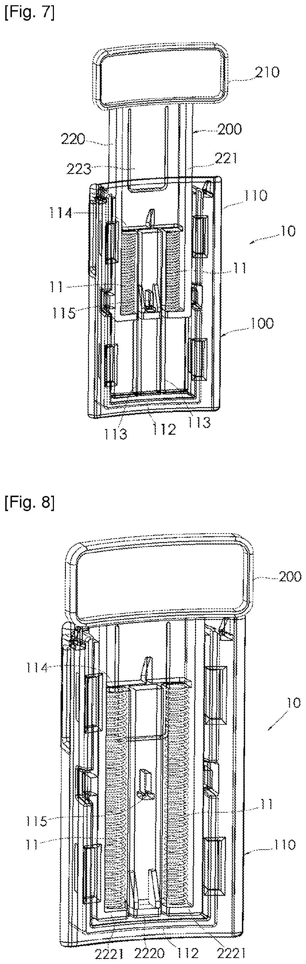

Ces deux parois latérales 221 s'étendent préférentiellement depuis la tête 210 du coulisseau 200 jusqu'à son extrémité interne au niveau de laquelle elles sont reliées l'une à l'autre par une paroi transversale d'extrémité 222, comme visible sur les figures 3 et 8.

Le terme « transversal » est relatif dans le présent texte à une direction perpendiculaire à une direction définie par l'axe longitudinale du fourreau 100 et du coulisseau 200.

Préférentiellement, les organes de guidage sont également formés par les flancs 111 du flasque intrados 110, chacune des parois latérales 221 du coulisseau 200 étant destinée à glisser contre l'une desdits flancs 111 du flasque intrados 110 lors du coulissement du coulisseau.

Avantageusement, les flancs 111 du flasque intrados 110 et les parois latérales 221 du coulisseau 200 sont dimensionnées de sorte à interdire tout débattement angulaire dudit coulisseau 200.

Les organes de guidage peuvent également comprendre, comme le représentent les figures 6, 7 et 8 dans un exemple préféré de réalisation, deux parois médianes longitudinales 113 parallèles s'étendant longitudinalement sur la face interne du flasque intrados 110, lesdites parois médianes longitudinales 113 étant agencées à distance l'une de l'autre de sorte à former une rainure médiane. A l'une de leurs extrémités, les parois médianes longitudinales 113 sont prolongées par la paroi de fond 112 du flasque intrados 110 et sont prolongées à l'autre extrémité par une paroi médiane transversale 114 du flasque intrados 110.

Afin de guider le coulissement du coulisseau 200 au sein du fourreau 100, la paroi transversale d'extrémité 222 comporte un évidement au droit de chacune des parois médianes longitudinales 113. Ainsi, et comme le montrent en particulier les figures 3 et 8, la paroi transversale d'extrémité 222 est divisée en trois portions : une portion médiane 2220 interposée entre deux portions latérales 2221.

L'attelle active 10 comporte des moyens de rappel élastique configurés pour solliciter le coulisseau 200 vers l'intérieur du fourreau 100, c'est à dire pour la rétracter.

Dans l'exemple représenté sur les figures 7 et 8, les moyens de rappel élastique sont formés par deux ressorts hélicoïdaux 11 travaillant en compression.

Dans d'autres exemples de réalisation, les moyens de rappel élastiques peuvent être formés par au moins un ressort hélicoïdal.

Chaque ressort hélicoïdal 11 prend appui par une de ses extrémités, contre l'une des portions latérales 2221 de la paroi transversale d'extrémité 222, et par son autre extrémité, contre la paroi de fond 112 du flasque intrados 110.

Comme le montrent les figures 7 et 8, chacun des ressorts hélicoïdaux 11 est avantageusement enfermé dans une cage formée par les organes de guidage, et donc par des nervures. Plus particulièrement, chaque cage est formée par la combinaison d'un flanc 111 du flasque intrados 110, d'une portion latérale 2221 de la paroi transversale d'extrémité 222, d'une paroi médiane longitudinale 113 et d'une partie de la paroi médiane transversale 114 du flasque intrados 110. Les ressorts hélicoïdaux 11 sont donc agencés de part et d'autre de la rainure médiane.

Pour entraîner le coulisseau 200 en position déployée, l'utilisateur doit donc aller à l'encontre de forces de rappel exercées par les ressorts hélicoïdaux 11.

Avantageusement, le flasque intrados 110 comporte une butée médiane 115 destinée à reposer contre la portion médiane 2220 de la paroi transversale d'extrémité 222 lorsque le coulisseau 200 est dans sa position déployée, comme le montre la figure 7. Cette butée médiane 115 a donc pour fonction de définir la course maximale du coulisseau 200 en extension.

La paroi de fond 112 du flasque intrados 110 constitue avantageusement une butée définissant la position rétractée du coulisseau 200, dans laquelle la paroi transversale d'extrémité 222 prend appui contre la paroi de fond 112 du flasque intrados 110.

L'attelle active 10 comporte un organe de blocage du coulisseau 200 adapté à maintenir ledit coulisseau 200 dans sa position déployée, comme il est représenté sur les figures 6 et 7.

Cet organe de blocage est configuré pour être automatiquement actionné lors du déplacement du coulisseau 200 vers sa position déployée, et plus précisément, lorsqu'il atteint sa position déployée.

À cet effet, comme le montrent notamment les figures 2, 3 et 6, le coulisseau 200 comporte une languette élastique 223 comprenant une excroissance 224 à son extrémité libre, ladite languette élastique 223 étant configurée de sorte que son excroissance 224 coopère avec une lumière 123 dont est pourvue le flasque extrados 120 sur sa face interne.

Dans l'exemple de réalisation représenté sur les figures 4 et 6, la lumière 123 est traversante.

En d'autres termes, l'excroissance 224 est adaptée à venir se loger dans ladite lumière 123 par déformation de la languette élastique 223 lorsque le coulisseau 200 occupe sa position déployée.

Avantageusement, le flasque extrados 120 comporte un renfoncement 124 sur sa face interne, sur une épaisseur correspondant au moins à l'épaisseur de l'excroissance 224 par rapport à la face extrados du coulisseau ; ledit renfoncement 124 étant agencée de sorte que, lorsque le coulisseau 200 est en position rétractée et sur une partie de sa course vers sa position déployée, l'excroissance 224 est logée au sein du renfoncement 124 et la languette élastique 223 est dans une position de repos, c'est-à-dire qu'elle ne subit aucune déformation.

Autrement dit, le renfoncement 124 est configuré de sorte à permettre de loger l'excroissance 224 de la languette élastique 223 lors d'une partie de son déplacement depuis la position rétractée du coulisseau 200 vers sa position déployée.

Préférentiellement, le renfoncement 124 et la lumière 123 sont séparés par un pont de matière 125 comme le montrent les figures 4 et 6.

Avantageusement, l'excroissance 224 présente une section droite comprise dans un plan parallèle à l'axe longitudinal du coulisseau 200, dont une portion 225 décroit jusqu'à la surface de la languette élastique 223, tel qu'illustré sur la figure 2.

Cette portion 225 décroissante constitue une surface d'appui contre le pont de matière 125 de sorte que lorsque le coulisseau 200 est entraîné vers sa position déployée, la languette élastique 223 est déformée progressivement.

Lors de la suite de la course du coulisseau 200 vers sa position déployée, l'excroissance 224 traverse le pont de matière 125 en glissant contre lui, la languette élastique 223 subissant alors un état de déformation maximale, jusqu'à ce que ladite excroissance 224 se trouve en vis-à-vis de la lumière 123 dans laquelle elle est forcée à se loger par l'effort généré par le retour élastique de ladite languette élastique 223, qui retrouve alors sa position de repos.

Cette déformation élastique participe à transmettre un retour tactile à un utilisateur avant et pendant le blocage du coulisseau 200 dans sa position déployée.

Afin de libérer le coulisseau 200 de sa position déployée, l'attelle active 10 comporte des moyens de détente adaptés à agir sur l'organe de blocage. Lorsqu'il n'est plus immobilisé en position déployée, le coulisseau 200 est entraîné vers sa position rétracté par l'effort de rappel généré par les ressorts hélicoïdaux 11.

Avantageusement, l'utilisateur actionne les moyens de détente pour générer un effort sur la tête du péroné contre lequel est destiné à reposer la tête 210 du coulisseau 200, afin de déplacer ladite tête du péroné vers sa position normalisée.

Avantageusement, dans l'exemple préféré de réalisation de l'invention, les moyens de détente agissent sur l'organe de blocage par la déformation élastique du fourreau 100 et du coulisseau 200, plus particulièrement de la languette élastique 223.

Comme représenté sur les figures 1, 4 et 6, le flasque extrados 120 comporte une partie en porte-à-faux 126 s'étendant au-delà du volume interne. Cette partie en porte-à-faux 126 est configurée pour être en regard de la languette élastique 223 lorsque le coulisseau 200 est en position déployée. La partie en porte-à-faux 126 présente avantageusement des propriétés élastiques, de sorte qu'en appliquant une force de poussée suffisante sur cette dernière, l'utilisateur peut entraîner sa déformation afin d'appliquer un effort de poussée sur la languette élastique 223 pour provoquer sa déformation et déloger l'excroissance 224 de la lumière 123.

Ainsi, l'utilisateur peut, par application d'une force de poussée sur la partie en porte-à-faux 126 du flasque externe, libérer le coulisseau 200 de sa position déployée et provoquer son déplacement vers sa position rétractée sous les forces de rappels générées par les ressorts hélicoïdaux 11.

Sur les figures 4 et 6, la lumière 123 est réalisée sur la partie en porte-à-faux 126. Toutefois, dans un autre exemple de réalisation de l'invention, la lumière 123 pourrait être réalisée à un autre endroit du flasque extrados 120 sans compromettre le fonctionnement précité de l'invention.

Avantageusement, la partie en porte-à-faux 126 peut comprendre un téton 127 hémisphérique comme visible sur la figure 4, ou tout autre type de saillie, s'étendant vers la languette élastique 223.

Ce téton 127 permet de concentrer les efforts appliqués par la partie en porte-à-faux 126 sur la languette élastique 223 et donc de réduire les frottements entre la partie en porte-à-faux 126 et ladite languette élastique 223, et permet ainsi de faciliter leur déformation.

Dans un autre exemple de réalisation (non représenté sur les figures), la partie en porte-à-faux 126 ne présente pas de propriété élastique et les moyens de détente sont formées par un bouton poussoir traversant ladite partie en porte-à-faux 126, ledit bouton poussoir étant configuré pour pouvoir appliquer une force de poussée sur la languette élastique 223 de sorte à déloger l'excroissance 224 de la lumière 123.

Dans cet exemple de réalisation, les moyens de détente agissent sur l'organe de blocage par déformation élastique du coulisseau 200 uniquement.

La présente invention s'intègre selon un autre aspect, dans un kit de traitement d'une subluxation du péroné.

Le kit comporte, en plus de l'attelle active 10, une attelle passive 20, destinée à être utilisée à la suite de l'utilisation de l'attelle active 10, en complément de cette dernière.

Plus particulièrement, l'attelle passive 20 décrite ci-après est prévue pour maintenir la tête du péroné dans sa position normalisée par l'attelle active 10 afin de modifier la mémoire des structures ligamentaires.

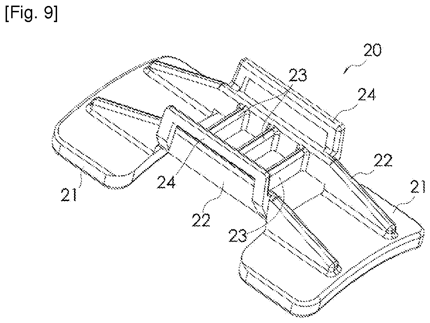

Comme le montre la figure 9 dans un exemple de réalisation, l'attelle passive 20 comporte deux zones d'appui 21 reliées entre elles par deux branches parallèles 22.

Ces branches parallèles sont jointes l'une à l'autre par des nervures transversales 23. Ces nervures transversales 23 permettent d'augmenter la résistance mécanique de l'attelle passive 20 tout en participant à la diminution de sa masse.

L'attelle passive 20 comporte en outre des boucles 24 destinées à recevoir un lien élastique (non représenté sur les figures) afin de maintenir ladite attelle passive 20, et plus particulièrement ses zones d'appui 21, contre la jambe de l'utilisateur.

L'ensemble des surfaces de l'attelle active 10 et de l'attelle passive 20 destinées au contact direct ou indirect avec les téguments d'un utilisateur peut avantageusement être recouverte d'un textile hypoallergénique.

Par ailleurs, le fourreau 100 et le coulisseau 200 de l'attelle active 10 ainsi que l'attelle passive 20 peuvent être réalisés en matériau thermo-formable.

De manière plus générale, il est à noter que les modes de mise en oeuvre et de réalisation de l'invention considérés ci-dessus ont été décrits à titre d'exemples non limitatifs et que d'autres variantes sont par conséquent envisageables.

## Revendications

1. Attelle active (10) comportant un fourreau (100) dans lequel un coulisseau (200) est agencé mobile en coulissement entre une position rétractée et une position déployée, le coulisseau (200) comprenant une tête (210) destinée à reposer en appui contre la tête d'un péroné d'un utilisateur, ladite attelle active (10) comprenant :
- un organe de blocage du coulisseau (200) adapté à maintenir ledit coulisseau (200) dans sa position déployée,
- des moyens de détente adaptés à agir sur l'organe de blocage de sorte à libérer le coulisseau (200) de sa position déployée,
l'attelle active étant **caractérisée en ce que** elle comporte des moyens de rappel élastique configurés pour solliciter le coulisseau (200) vers sa position rétractée.

2. Attelle active (10) selon la revendication 1, dans laquelle les moyens de détente agissent sur l'organe de blocage par la déformation élastique du fourreau (100) et du coulisseau (200).

3. Attelle active (10) selon l'une des revendications 1 ou 2, dans laquelle l'organe de blocage comprend une languette élastique (223) formée dans le coulisseau (200), comportant à une extrémité libre une excroissance (224) adaptée à se loger dans une lumière (123) réalisée dans le fourreau (100).

4. Attelle active (10) selon la revendication 3, dans laquelle le fourreau (100) comporte un renfoncement (124) s'étendant en vis-à-vis du coulisseau (200), ledit renfoncement (124) étant configuré pour recevoir l'excroissance (224) lorsque le coulisseau (200) est en position rétractée et sur une partie de sa course vers sa position déployée, le renfoncement (124) et la lumière (123) étant séparés par un pont de matière (125).

5. Attelle active (10) selon l'une des revendications 3 ou 4, dans laquelle les moyens de détente sont formés par une partie en porte-à-faux (126) du fourreau (100), s'étendant au-delà de la lumière (123), configurée pour être en regard de la languette élastique (223) lorsque le coulisseau (200) est en position déployée et présentant des propriétés élastiques.

6. Attelle active (10) selon l'une des revendications 1 à 5, dans laquelle le fourreau (100) comporte un flasque intrados (110) destiné à être en appui contre un membre inférieur d'un utilisateur, et un flasque extrados (120) opposé au flasque intrados (110), lesdits flasques étant assemblés l'un avec l'autre et étant configurés pour définir un volume interne dans lequel est engagé le coulisseau (200).

7. Attelle active (10) selon l'une des revendications 1 à 6, comportant des organes de guidage permettant le guidage du coulisseau (200) en translation au sein du fourreau (100), lesdits organes de guidage étant formés par des nervures s'étendant sur le coulisseau (200) et sur le fourreau (100) et coopérant les unes avec les autres.

8. Attelle active (10) selon la revendication 7, dans laquelle les moyens de rappel élastique sont formés par au moins un ressort hélicoïdal (11) enfermé dans une cage formée par les organes de guidage.

9. Kit comportant une attelle active (10) selon l'une des revendications 1 à 8 et une attelle passive (20) destinée à maintenir la tête du péroné dans une position normalisée, ladite attelle passive comportant :
- deux zones d'appui reliées entre elles par un pont de matière (125), une des zones d'appui étant destinée à être appliquée contre ladite tête du péroné et l'autre zone d'appui étant destinée à être appliquée contre le fut osseux du péroné,
- un lien élastique destinée à maintenir un effort appliqué par lesdites zones d'appui contre le membre inférieur d'un l'utilisateur.

## Patentansprüche

1. Aktive Schiene (10), umfassend eine Hülle (100), in der einen Schieber (200) gleitbar zwischen einer zurückgezogenen Position und einer ausgefahrenen Position angeordnet ist, wobei der Schieber (200) einen Kopf (210) umfasst, der dazu bestimmt ist, gegen den Kopf eines Wadenbeins eines Benutzers zu ruhen, wobei die aktive Schiene (10) Folgendes umfasst:
- ein Blockierelement des Schiebers (200), das angepasst ist, um den Schieber (200) in seiner ausgefahrenen Position aufrechtzuerhalten,
- Entlastungsmittel, die angepasst sind, um auf das Blockierelement einzuwirken, um den Schieber (200) aus seiner ausgefahrenen Position freizugeben, wobei die aktive Schiene **dadurch gekennzeichnet ist, dass** sie elastische Rückstellmittel umfasst, die so konfiguriert sind, dass sie den Schieber (200) in Richtung seiner zurückgezogenen Position vorspannen.

2. Aktive Schiene (10) nach Anspruch 1, wobei die Entlastungsmittel auf das Blockierelement durch die elastische Verformung der Hülle (100) und des Schiebers (200) wirken.

3. Aktive Schiene (10) nach einem der Ansprüche 1 oder 2, wobei das Blockierelement eine elastische Zunge (223) umfasst, die in dem Schieber (200) ausgebildet ist, und an einem freien Ende einen Vorsprung (224) umfasst, der angepasst ist, um in einer Öffnung (123) aufgenommen zu werden, die in der Hülle (100) hergestellt ist.

4. Aktive Schiene (10) nach Anspruch 3, wobei die Hülle (100) eine Aussparung (124) umfasst, die sich gegenüber dem Schieber (200) erstreckt, wobei die Aussparung (124) so konfiguriert ist, dass sie den Vorsprung (224) aufnimmt, wenn sich der Schieber (200) in der zurückgezogenen Position und über einen Teil seiner Bewegung nach seiner ausgefahrenen Position befindet, wobei die Aussparung (124) und die Öffnung (123) durch eine Materialbrücke (125) getrennt sind.

5. Aktive Schiene (10) nach einem der Ansprüche 3 oder 4, wobei die Entlastungsmittel durch einen freitragenden Abschnitt (126) der Hülle (100) gebildet werden, der sich über die Öffnung (123) hinaus erstreckt, so konfiguriert ist, dass er der elastischen Zunge (223) zugewandt ist, wenn sich der Schieber (200) in der ausgefahrenen Position befindet, und elastische Eigenschaften aufweist.

6. Aktive Schiene (10) nach einem der Ansprüche 1 bis 5, wobei die Hülle (100) einen Flansch mit innerer Laibung (110), der bestimmt ist, um gegen eine untere Extremität eines Benutzers anzuliegen, und einen Flansch mit äußerer Laibung (120) umfasst, der dem Flansch mit innerer Laibung (110) entgegengesetzt ist, wobei die Flansche zusammengebaut und so konfiguriert sind, dass sie ein Innenvolumen definieren, in das der Schieber (200) eingreift.

7. Aktive Schiene (10) nach einem der Ansprüche 1 bis 6, umfassend Führungselemente, die die Führung des Schiebers (200) in Translation innerhalb der Hülle (100) ermöglichen, wobei die Führungselemente durch Rippen gebildet sind, die sich über den Schieber (200) und über die Hülle (100) erstrecken und miteinander zusammenwirken.

8. Aktive Schiene (10) nach Anspruch 7, wobei die elastischen Rückstellmittel durch mindestens eine Schraubenfeder (11) gebildet sind, die in einem von den Führungselementen gebildeten Käfig eingeschlossen ist.

9. Kit, umfassend eine aktive Schiene (10) nach einem der Ansprüche 1 bis 8 und eine passive Schiene (20), die dazu bestimmt ist, den Kopf des Wadenbeins in einer normalisierten Position zu halten, wobei die passive Schiene Folgendes umfasst:
- zwei Stützzonen, die durch eine Materialbrücke (125) miteinander verbunden sind, wobei eine der Stützzonen dazu bestimmt ist, gegen den Kopf des Wadenbeins angelegt zu werden, und die andere Stützzone dazu bestimmt ist, gegen den Wadenbeinknochen angelegt zu werden,
- eine elastische Verbindung, die dazu bestimmt ist, eine Kraft aufrechtzuerhalten, die von den Stützzonen gegen die untere Extremität eines Benutzers ausgeübt wird.

## Claims

1. An active splint (10) comprising a sheath (100) within which a slider (200) is arranged so as to slidably move between a retracted position and a deployed position, the slider (200) comprising a head (210) intended to bear against the head of a perineal bone of a user, said active splint (10) comprising:
- a member for blocking the slider (200) adapted to hold said slider (200) in its deployed position,
- detent means adapted to act on the blocking member so as to release the slider (200) from its deployed position,
the active splint being **characterized in that** it comprises elastic return means configured to urge the slider (200) towards its retracted position.

2. The active splint (10) according to claim 1, wherein the detent means act on the blocking member by the elastic deformation of the sheath (100) and of the slider (200).

3. The active splint (10) according to one of claims 1 or 2, wherein the blocking member comprises an elastic tab (223) formed in the slider (200), comprising at one free end an excrescence (224) adapted to fit in an aperture (123) formed in the sheath (100).

4. The active splint (10) according to claim 3, wherein the sheath (100) includes a recess (124) extending opposite the slider (200), said recess (124) being configured to receive the excrescence (224) when the slider (200) is in the retracted position and over part of its stroke towards its deployed position, the recess (124) and the aperture (123) being separated by a bridge of material (125).

5. The active splint (10) according to one of claims 3 or 4, wherein the detent means are formed by a cantilevered portion (126) of the sheath (100), extending beyond the aperture (123), configured to face the elastic tab (223) when the slider (200) is in the deployed position and having elastic properties.

6. The active splint (10) according to one of claims 1 to 5, wherein the sheath (100) includes an intrados flange (110) intended to bear against a lower limb of a user, and an extrados flange (120) opposite to the intrados flange (110), said flanges being assembled together and being configured to define an inner volume in which the slider (200) is engaged.

7. The active splint (10) according to one of claims 1 to 6, including guide members enabling the translational guidance of the slider (200) within the sheath (100), said guide members being formed by ribs extending on the slider (200) and on the sheath (100) and cooperating with each other.

8. The active splint (10) according to claim 7, wherein the elastic return means are formed by at least one helical spring (11) enclosed in a cage formed by the guide members.

9. A kit including an active splint (10) according to one of claims 1 to 8 and a passive splint (20) intended to hold the head of the perineal bone in a normal position, said passive splint including:
- two support areas connected together by a bridge of material (125), one of the support areas being intended to be applied against said head of the perineal bone and the other support area being intended to be applied against the bone shaft of the perineal bone,
- an elastic link intended to maintain a force applied by said support areas against the lower limb of a user.
